(19) Europäisches Patentamt
European Patent Office
Office européen des brevets

(11) Publication number: **0 498 361 A2**

# (12) EUROPEAN PATENT APPLICATION

(21) Application number: **92101797.6**

(51) Int. Cl.$^5$: **A61K 37/64**

(22) Date of filing: **04.02.92**

(30) Priority: **06.02.91 US 651684**

(43) Date of publication of application:
**12.08.92 Bulletin 92/33**

(84) Designated Contracting States:
**PT**

(71) Applicant: **SCHERING CORPORATION**
**2000 Galloping Hill Road**
**Kenilworth New Jersey 07033(US)**

(72) Inventor: **Darrow, William R.**
**42 Palmerston Place**
**Basking Ridge, New Jersey 07920(US)**
Inventor: **Sybertz, Edmund J, Jr.**
**10 Ryan Court, Rd No 2**
**Chester, New Jersey 07930(US)**

(74) Representative: **von Kreisler, Alek, Dipl.-Chem. et al**
**Patentanwälte Von Kreisler-Selting-Werner,**
**Deichmannhaus am Hauptbahnhof**
**W-5000 Köln 1(DE)**

## (54) Combination of an angiotensin II antagonist or renin inhibitor with a neutral endopeptidase inhibitor.

(57) Treatment of hypertension or congestive heart failure with a combination of an angiotensin II antagonist or a renin inhibitor with a neutral endopeptidase inhibitor, pharmaceutical compositions comprising said combinations and kits for administering separate pharmaceutical compositions in combination are disclosed, wherein the angiotensin II antagonists include saralasin, sar 1, ile 8 angiotensin II, Dup 753, EXP 6155, EXP 6803 and PD 123319, the renin inhibitors include enalkrein, RO 42-5892, A 65317, CP 80794, ES 1005, ES 8891, SQ 34017, CGP 29287, CGP 38560, SR 43845, U-71038, A 62198, and A 64662, and the neutral endopeptidase inhibitors include N-[N-[1(S)-carboxyl-3-phenylpropyl]-(S)-phenylalanyl]-(S)-isoserine, N-[N-[((1S)-carboxy-2-phenyl)ethyl]-(S)-phenylalanyl]-$\beta$-alanine; N-[2(S)-mercaptomethyl-3-(2-methylphenyl)-propionyl]methionine, SQ 28603, UK 69578, SQ 29072, thiorphan, retro-thiorphan and phosphoramidon.

EP 0 498 361 A2

## BACKGROUND OF THE INVENTION

The present invention relates to the treatment of hypertension and congestive heart failure with a combination of an angiotensin II antagonist or a renin inhibitor with a neutral endopeptidase inhibitor.

In a second aspect, this invention relates to a pharmaceutical composition comprising an A II antagonist or a renin inhibitor in combination with an NEP inhibitor and to kits comprising an A II antagonist and an NEP inhibitor or a renin inhibitor and an NEP inhibitor.

The renin angiotensin system is a complex hormonal system comprised of a large molecular weight precursor, angiotensinogen, two processing enzymes, renin and angiotensin converting enzyme (ACE), and a vasoactive mediator, A II. See J. Cardiovasc. Pharmacol., 15(Supp B) (1990) p. S1-S5. The enzyme renin catalyzes the cleavage of angiotensinogen into the decapeptide angiotensin I, which has minimal biological activity on its own and is converted into the active octapeptide A II by ACE. A II has multiple biological actions on the cardiovascular system, including vasoconstriction, activation of the sympathetic nervous system, stimulation of aldosterone production, antinatriuresis, stimulation of vascular growth and stimulation of cardiac growth. A II functions as a pressor hormone and is involved the pathophysiology of several forms of hypertension.

Inhibitors of the renin angiotensin system are well known; such drugs lower blood pressure and exert beneficial actions in hypertension and in congestive heart failure as described, for example, in N. Eng. J. Med.. 316, 23 (1987) p. 1429-1435. A large number of peptide and non-peptide inhibitors of the renin angiotensin system are known, the most widely studied being the ACE inhibitors, which class includes the drugs captopril, enalapril, lisinopril and spirapril. Although a major mode of action of ACE inhibitors involves prevention of formation of the vasoconstrictor peptide A II, it has been reported in Hypertension, 16, 4 (1990) p. 363-370 that ACE cleaves a variety of peptide substrates, including the vasoactive peptides bradykinin and substance P. Prevention of the degradation of bradykinin by ACE inhibitors has been demonstrated, and the activity of the ACE inhibitors in some conditions has been reported in Circ. Res., 66, 1 (1990) p. 242-248 to be mediated by elevation of bradykinin levels rather than inhibition of A II formation. Consequently, it cannot be presumed that the effect of an ACE inhibitor is due solely to prevention of angiotensin formation and subsequent inhibition of the renin angiotensin system.

Neutral endopeptidase (EC 3.4.24.11; enkephalinase; atriopeptidase; NEP) is a zinc-containing metalloprotease which cleaves a variety of peptide substrates on the amino terminal side of aromatic amino acids. See Biochem. J., 241, (1987) p. 237-247. Substrates for this enzyme include, but are not limited to, atrial natriuretic factors (ANF), brain natriuretic peptide, met and leu enkephalin, bradykinin, neurokinin A, and substance P.

ANF are a family of vasodilator, diuretic and antihypertensive peptides which have been the subject of many recent reports in the literature, for example Annu. Rev. Pharm. Tox., 29, (1989) p. 23-54. One form, ANF 99-126, is a circulating peptide hormone which is released from the heart during conditions of cardiac distension. The function of ANF is to maintain salt and water homeostasis as well as to regulate blood pressure. ANF is rapidly inactivated in the circulation by at least two processes: a receptor-mediated clearance reported in Am. J. Physiol., 256 (1989) p. R469-R475 and an enzymatic inactivation via NEP reported in Biochem. J., 243 (1987) p. 183-187. It has been previously demonstrated that inhibitors of NEP potentiate the hypotensive, diuretic, natriuretic and plasma ANF responses to pharmacological injection of ANF in experimental animals. The potentiation of ANF by two specific NEP inhibitors is reported by Sybertz et al in J. Pharmacol. Exp. Ther.. 250. 2 (1989) p. 624-631 and in Hypertension, 15, 2 (1990) p. 152-161, while the potentiation of ANF by NEP in general was disclosed in U.S. patent 4,749,688. In U.S. 4,740,499, Olins disclosed the use of thiorphan and kelatorphan to potentiate atrial peptides. Moreover, NEP inhibitors lower blood pressure and exert ANF-like effects such as diuresis and increased cyclic guanosine 3',5'-monophosphate (cGMP) excretion in some forms of experimental hypertension. The antihypertensive action of NEP inhibitors is mediated through ANF because antibodies to ANF will neutralize the reduction in blood pressure.

U.S. 4,749,688 also established the antihypertensive action of NEP inhibitors and that co-administration of an ACE inhibitor and a NEP inhibitor results in a greater reduction of blood pressure than observed with either agent alone. The antihypertensive effect is best manifested under conditions in which the renin angiotensin system is suppressed, as reported by Sybertz et al in the references cited above. For example, NEP inhibitors reduce blood pressure effectively in the Desoxycorticosterone sodium acetate (DOCA NA) hypertensive rat, a volume-dependent, renin-suppressed model of hypertension, but are less effective under conditions in which the renin angiotensin system is activated, such as in the spontaneously hypertensive rat (SHR) and in the two kidney Goldblatt hypertension model. Studies in the SHR and in the two-kidney Goldblatt hypertension model using a prodrug of the NEP inhibitor N-[2(S)-mercaptomethyl-3-(2-methylphenyl)propionyl]methionine in combination with the ACE inhibitor spirapril demonstrated the greater efficacy of the combination compared to either drug alone. However, this interaction was inhibited in SHR which had been nephrectomized, a manipulation which markedly suppresses renin levels.

An explanation of this interactive effect of ACE inhibitors and NEP inhibitors on blood pressure is that suppression of the renin angiotensin system allows for full expression of the ANF-like antihypertensive effect of the NEP inhibitor. A II and ANF exert opposite effects on the cardiovascular system and it has been proposed by Johnston et al in Am. J. Med., 87, (Supp 6) (1990) p. 6B-24S-6B-28S that these two hormonal systems act to counterbalance one another.

An enhanced effect from a combination of an A II receptor antagonist or a renin inhibitor with an NEP inhibitor is, however, unexpected for several reasons. First, as discussed above, ACE inhibitors exert pharmacological effects other than inhibition of formation of A II. ACE degrades numerous substrates, including bradykinin, neurotensin, and substance P. In some instances, e.g. with bradykinin and substance P, both ACE and NEP will degrade the peptide. Since substance P and bradykinin are vasodilators, an alteration of the metabolism of either of these, or more efficient protection from degradation by inhibiting the two enzymes could account for an enhanced effect. Moreover, although nephrectomy, a maneuver which strikingly reduces plasma renin levels, eliminated the enhanced interaction of the ACE inhibitor and NEP inhibitor, the NEP inhibitor alone did not lower blood pressure in this state. Thus, the interactions of ACE inhibitors and NEP inhibitors are complex and the effect of an A II receptor antagonist or a renin inhibitor in combination with an NEP inhibitor cannot be predicted solely from data obtained from the combination of an ACE inhibitor and NEP inhibitor.

## SUMMARY OF THE INVENTION

The present invention relates to a method of treating hypertension or congestive heart failure comprising administering an effective amount of a combination of an A II antagonist and a NEP inhibitor to a mammal in need of such treatment. The invention also relates to a method of treating hypertension or congestive heart failure comprising administering an effective amount of a combination of a renin inhibitor and a NEP inhibitor to a mammal in need of such treatment.

Another aspect of the invention relates to pharmaceutical compositions comprising an effective amount of a combination of an A II antagonist and a NEP inhibitor in a pharmaceutically acceptable carrier and to pharmaceutical compositions comprising an effective amount of a combination of a renin inhibitor and a NEP inhibitor in a pharmaceutically acceptable carrier.

Since the present invention relates to a method of treatment comprising a combination of actives wherein the actives may be administered separately, the invention in a third aspect relates to combining separate pharmaceutical compositions in kit form.

## DETAILED DESCRIPTION

The NEP inhibitors suitable for use in this invention include, but are not limited to compounds disclosed in U.S. 4,610,816, herein incorporated by reference, including in particular N-[N-[1(S)-carboxyl-3-phenylpropyl]-(S)-phenylalanyl]-(S)-isoserine and N-[N-[((1S)-carboxy-2-phenyl)ethyl]-(S)-phenylalanyl]-β-alanine; compounds disclosed in U.S 4,801,609 and 4,929,641, each herein incorporated by reference, including compounds of the formula

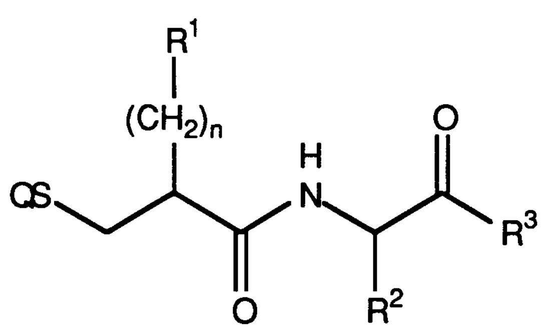

wherein $R^1$ is phenyl substituted by alkyl, $R^2$ is alkyl-$S(O)_{0\text{-}2}(CH_2)q$, $R^3$ is $OR^7$ wherein $R^7$ is hydrogen or lower alkyl, Q is hydrogen or $R^{10}CO$- wherein $R^{10}$ is alkyl, n is 0-2 and q is 1-4, and in particular N-[2(S)-mercaptomethyl-3-(2-methylphenyl)-propionyl]methionine; SQ 28603 (N-[2-(mercaptomethyl)-1-oxo-3-phenylpropyl]-β-alanine), disclosed in South African Patent Application 84/0670; UK 69578 (cis-4-[[[1-[2-carboxy-3-(2-methoxyethoxy)propyl]-cyclopentyl]carbonyl]amino]-cyclohexanecarboxylic acid) and its active enantiomer(s); thiorphan and its enantiomers; retro-thiorphan; phosphoramidon; and SQ 29072 (7-[[2-(mercaptomethyl)-1-oxo-3-phenylpropyl]amino]-heptanoic acid). Also suitable for use are any pro-drug forms of the above-listed NEP inhibitors, e.g., compounds in which one or more carboxylic acid groups are esterified.

The A II antagonists suitable for use in this invention include, but are not limited to saralasin; sar 1 (1-(N-methylglycine-angiotensin II); ile 8 angiotensin II (1-de-L-aspartic acid -8-L-isoleucine-angiotensin II); Dup 753 (2-butyl-4-chloro-1-[[2'-(1H-tetrazol-5-yl)[1,1'-biphenyl]-4-yl]methyl]-1H-imidazole-5-methanol, monopotassium salt) and active metabolites thereof; EXP 6155 ( 2-butyl-1-[(4-carboxyphenyl)methyl]-4-chloro-1H-imidazole-5-acetic acid, disodium salt); EXP 6803 (2-butyl-1-[[4-[(2-carboxybenzoyl)amino]phenyl]-methyl]-4-chloro-1H-imidazole-5-acetic acid α-methyl ester, monosodium salt); and PD 123319 (1-(4-dimethylamino-3-methylphenyl)methyl-5-diphenylacetyl-4,5,6,7-tetrahydro-1H-imidazo[4,5-c]pyridine-6-carboxylic acid]. Dup 753,EXP 6155 and EXP 6803 are disclosed in European Patent Applications 253,310 and 324,377; PD 123319 is disclosed in European Patent Application 245,637.

The renin inhibitors suitable for use in the present invention include, but are not limited to, enalkrein, RO 42-5892, A 65317 [(2R)-2-benzyl-3-[(2-methoxyethoxymethoxyethyl)methylaminocarbonyl]-propionyl-L-His(2'S,1'R,5S)-3-ethyl-5(1'-hydroxy-2'-amino-3'-cyclohexylpropyl)oxazolidin-2-one amide]; CP 80794; ES 1005 (N-[4-[[1-[[(5-amino-6-hydroxyhexyl)-amino]carbonyll]-3-methyl-butyl]amino]-2-hydroxy-1-(2-methylpropyl)-4-oxobutyl]-α-[[3-(1-naphthalenyl)-2-(1-naphthalenylmethyl)-1-oxopropyl]-amino]-1H-imidazole-4-propanamide dihydrochloride); ES 8891 (5-cyclohexyl-2,4,5-trideoxy-N-hexyl-4-[[N-[3-(1-naphthalenyl)-N-(-4-morpholinyl-acetyl)-L-alanyl]-3-(4-thiazolyl)-L-alanyl]amino]-L-threo-pentonamide); SQ 34017; CGP 29287 (carbobenzyloxy-Arg-Arg-Pro-Phe-His-Sta-Ile-His-Lys(BOC)OMe); CGP 38560 (N-[4-[(butylamino)-carbonyl]-1-(cyclohexylmethyl)-2-hydroxy-5-methylhexyl]-α-[[2-[[(1,1-dimethylethyl)-sulfonyl]methyl]-1-oxo-3-phenylpropyl]amino]-1H-imidazole-4-propanamide), disclosed in U.S. 4,758,584; SR 43845 (3-Pyr-($CH_2$)-CO-Phe-His-ACHPA-Ile-NH-C($CH_3$-($CH_2OH$)$_2$); U-71038 (BOC-Pro-Phe-N-MeHis-Leuψ($CHOHCH_2$)Val-Ile-Amp); A 62198 ([N-(2-methyl-1-oxopropyl)-L-phenylalanyl]-N-[1S,2R,3S]-4-azido-1-(cyclohexylmethyl)-2,3-dihydroxybutyl-L-histidinamide); A 64662 ([N-(3-amino-3-methyl-1-oxobutyl)-4-methoxy-L-phenylalanyl]-N-[1S,2R,3S]-1-(cyclohexyl-methyl)-2,3-dihydroxy-5-methylhexyl-L-histidinamide)and those disclosed by Watkins et al in U.S. 4,906,613, including those disclosed in the following publications, cited therein:

Compounds of the formula

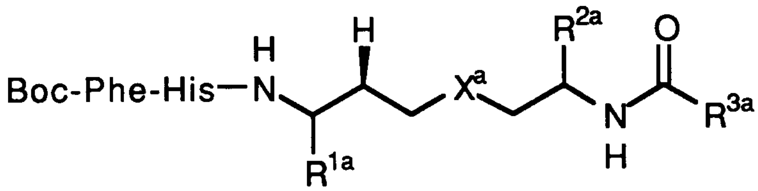

wherein $R^{1a}$ is selected from cyclohexylmethyl, benzyl or butyl; $X^a$ is S or O; $R^{2a}$ is selected from isobutyl, cyclohexylmethyl or benzyl; and $R^{3a}$ is phenethyl. A preferred compound within this class is one wherein $R^{1a}$ is cyclohexylmethyl, $R^{2a}$ is isobutyl, $R^{3a}$ is phenethyl and $X^a$ is S. These compounds are described by Luly et al., Pharmacologist, 27 (3), (1985) p. 260, , and can be prepared by known techniques from known materials.

Other renin inhibitors are disclosed, for example Szelke et al., U.S. Pat. No. 4,424,207 discloses as having the formula

$X^b$-$Y^b$-Pro-Phe-His-$A^b$-$B^b$-$Z^b$-$W^b$ (V)

where

Pro, Phe and His may be in substituted form;

$X^b$ = H; or an acyl or other N-protecting group e.g. acetyl, pivaloyl, t-butyloxycarbonyl (Boc), benzoyl or lower alkyl (primarily $C_1$-$C_5$); or an L- or D-amino-acyl residue, which may itself be N-protected similarly;

$Y^b$ = D- or L-His or other D- or L-basic or aromatic amino-acetyl residue, or is absent;

$A^b$ =

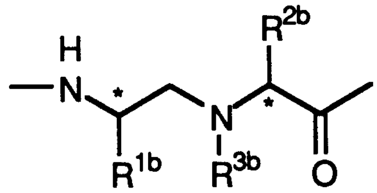

"reduced" isostere bond (VI)

or

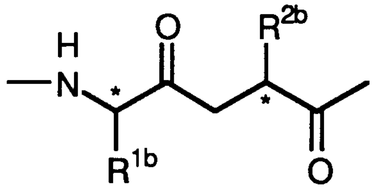

"keto" isostere bond (VII)

or

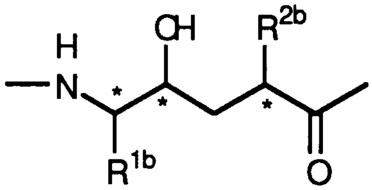

"hydroxy" isostere bond (VIII)

or

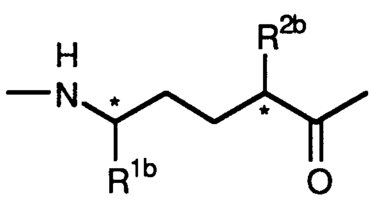

"hydrocarbon" isostere bond (IX)

where the configuration at asymmetric centers * is either R or S, where in VIII the hydroxy group may be present as such or protected in ether $-OR^{4b}$ or ester

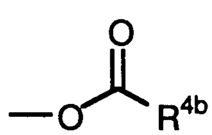

form where $R^{4b}$ is as given under W below and where $R^{1b}$ and $R^{2b}$, the same or different = $^1$Pro(isopropyl), $^1$Bu(isobutyl), Bz(benzyl) or other lipophilic or aromatic amino-acid side chain;

$R^{3b}$ = -H; lower alkyl ($C_1$-$C_5$); or $-SO_2Ph$, $-SO_2C_6H_4CH_3$(p), Boc, formyl or other N-protecting group;

$B^b$ = D- or L-Val or Ile or other D- or L-lipophilic aminoacyl residue;

$Z^b$ = D- or L-Tyr, Phe, His or other D-or L-aromatic aminoacyl residue; and

$W^b$ =

(a) -OH

(b) $-OR^{4b}$ where $R^{4b}$ = (1), lower alkyl $C_1$-$C_5$($n^1$), cycloalkyl $C_3$-$C_7$ or Bzi

(c) $-NH_2$

(d) $-NHR^{3b}$ or $-N(R^{5b})_2$ wherein $R^{5b}$ is an N-protecting group or $R^{4b}$

(e) L- or D-Lys
(f) L- or D-Arg unprotected or as the ester or amide
(g) L- or D-Ser and
(h) amino alcohol derived from (e)-(g) as such or protected in ester or ether form
$Z^b + W^b$ = alcohol derived from
(i) L-Tyr
(ii) L-Phe
(iii) D-Tyr or D-Phe
(iv) His

such peptide being in the above form or modified by isosteric replacement of one or more remaining peptide bonds by reduced, $-CH_2-NH-$, keto,

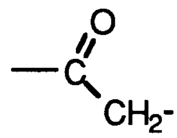

hydroxy, $-CH(OH)-CH_2-$, or hydrocarbon, $-CH_2-CH_2-$ isosteric links and further being in free form or in protected or salt form at one or more remaining peptide, carboxyl, amino, hydroxy or other reactive groups, in particular as their physiologically acceptable acid addition salts at basic centers.

Veber et al., U.S. 4,479,941, discloses renin inhibitor compounds such as
IBU-His-Pro-Phe-His-Sta-Leu-benzylamide;
IBU-His-Pro-Phe-His-Sta-Leu-2-phenylethylamide;
IBU-His-Pro-Phe-His-Sta-Leu-3-phenylpropylamide;
IBU-His-Pro-Phe-His-Sta-Leu-1,2-diphenylethylamide;
BOC-Phe-His-Sta-Leu-(+)[1]-1,2-diphenylethylamide;
BOC-Phe-His-Sta-Leu-(-)-1,2-diphenylethylamide;
BOC-Phe-His-Sta-Leu-benzylamide;
BOC-Phe-His-Sta-Leu-(+)-α-phenylethylamide;
BOC-Phe-His-Sta-Leu-(-)-α-phenylethylamide;
BOC-Phe-His-Sta-Leu-(+)-α-naphthylethylamide;
BOC-Phe-His-Sta-Leu-(-)-α-naphthylethylamide;
BOC-Phe-His-Sta-Leu-p-chlorobenzylamide;
BOC-Phe-His-Sta-Leu-p-methoxybenzylamide;
BOC-Phe-His-Sta-Leu-10,11-dihydro-5H-dibenzo[a,d]-cyclohepteneamide;
BOC-Phe-His-Sta-Leu-D,L-threo-1,2-diphenyl-2-hydroxyethylamide;
BOC-Phe-His-Sta-Leu-Sta;
BOC-Phe-His-AHPPA-Leu-benzylamide;
Acetyl-Phe-His-AHPPA-Leu-benzylamide;
BOC-Phe-His-Sta-Leu-(2-amidomethylpyridine);
BOC-Phe-His-Sta-Leu-(4-amidomethylpyridine);
BOC-Phe-His-Sta-Leu-(4-amido-1-benzylpiperidine);
BOC-Phe-His-Sta-Leu-[N-(3-amidopropyl)diethanolamine];
BOC-Phe-His-AHPPA-Leu-(2-amidomethylpyridine);
BOC-Phe-His-ACHPA-Ile-(2-amidomethylpyridine);
IVA-His-D-Pro-Phe-His-ACHPA-Ile-(2-amidomethylpyridine);

[1] (+) refers to the optical rotation of the amine.

A preferred compound within this class is BOC-Phe-His-Sta-Leu-(4-amido-1-benzyl-piperidine).

Veber et al., U.S. 4,478,826, discloses renin inhibitor compounds such as
tert-Butyloxycarbonyl-His-Pro-Phe-His-Sta-Leu-Leu-Leu-$OCH_3$,
tert-Butyloxycarbonyl-His-Pro-Phe-His-Sta-Leu-Tyr-$NH_2$,
iso-Butyryl-His-Pro-Phe-His-Sta-Leu-Phe-Lys-$NH_2$,
tert-Butyloxycarbonyl-His-Pro-Phe-p-I-Phe-Sta-Leu-Phe-$NH_2$,
iso-Valeryl-His-Pro-Phe-His-Sta-Leu-Val-Phe-$NH_2$,
His-Pro-Phe-His-Sta-Leu-Phe-$NH_2$,
iso-Valeryl-His-Pro-Phe-His-Sta-Leu-Phe-$NH_2$,
Acetyl-Pro-Phe-His-Sta-Leu-Phe-$NH_2$,

Acetyl-Phe-His-Sta-Leu-Phe-$NH_2$,
tert-Butyloxycarbonyl-Phe-His-Sta-Leu-Phe-$NH_2$,
tert-Butyloxycarbonyl-His-Pro-Phe-Phe-Sta-Leu-Phe-$NH_2$,
iso-Butyryl-His-Pro-Phe-His-Sta-Ala-Phe-$NH_2$,

| | | |
|---|---|---|
| iso-Butyryl-His-Pro-Phe-His-Sta | { | Cyclo-hexyl-Phe-$NH_2$<br>Ala |

A preferred compound within this class is IVA-His-Pro-Phe-His-Sta-Leu-Phe-$NH_2$.
Veber et al., U.S. 4,384,994 discloses renin inhibitor compounds such as
N-phenoxyacetyl-L-leucyl-(3S,4S)-statyl-L-valyl-L-phenylalanine;
N-phenoxyacetyl-L-leucyl-(3S,4S)-statyl-L-leucyl-L-phenylalanine
N-phenoxyacetyl-L-leucyl-(4S)-amino-(3S)-hydroxy-5-phenylpentanoyl-L-leucyl-L-phenylalanine;
L-leucyl-(3S,4S)-statyl-L-valyl-L-phenylalanine;
L-leucyl-(3S,4S)-statyl-L-leucyl-L-phenylalanine;
L-leucyl-(4S)-amino-(3S)-hydroxy-5-phenylpentanoyl-L-leucyl-L-phenylalanine;
and the amide and $C_{1-4}$ alkyl ester forms of the above peptides.
Boger et al., U.S. 4,485,099, discloses renin inhibitor compounds such as

IBU-His-Pro-Phe—Lys-Sta-Leu-Phe

IBU-His-Pro-Phe—Orn-Sta-Leu-Phe

IBU-His-Pro-Phe—DAB-Sta-Leu-Phe-Gly

IBU-His-Pro-Phe—HLys-Sta-Leu-Phe

IBU-His-Pro-Phe—Orn-Sta-Leu-Phe-Gly

IBU-His-Pro-Phe—Lys-Sta-Leu-Phe-Gly

BOC-Phe—Lys-Sta-Leu-Phe

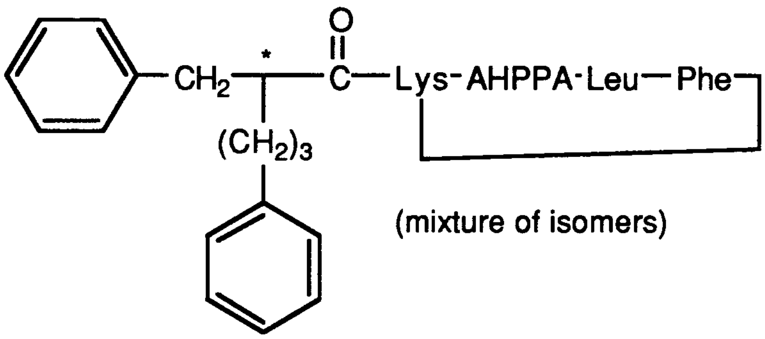

(mixture of isomers)

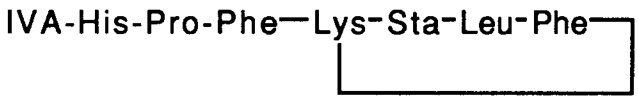

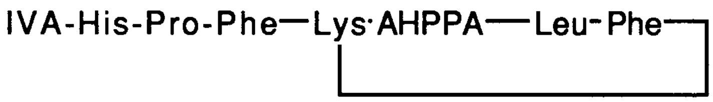

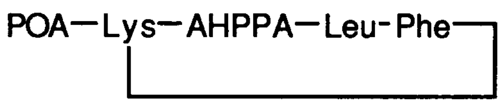

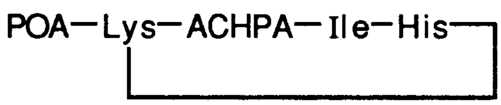

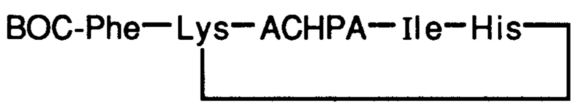

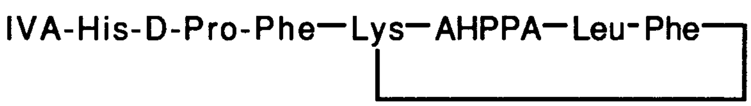

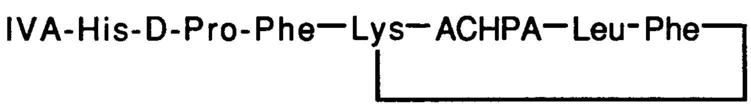

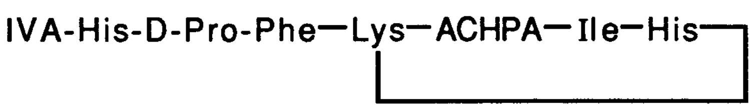

A preferred compound within this class is

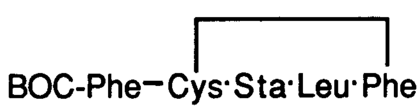

Boger et al., U.S. 4,477,441, discloses renin inhibitor compounds such as

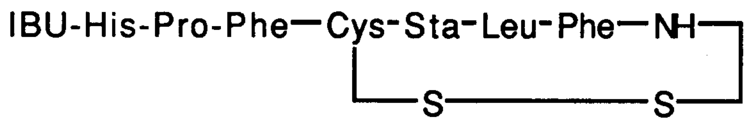

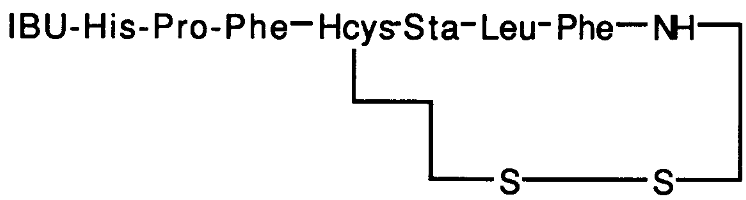

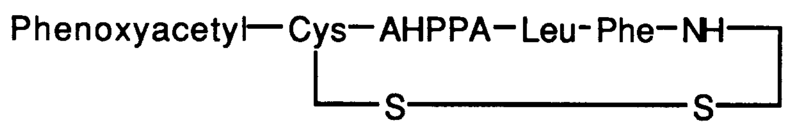

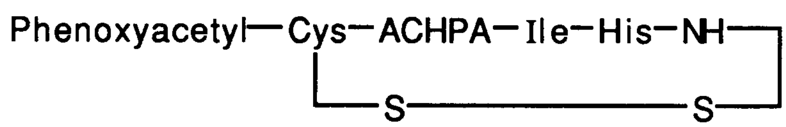

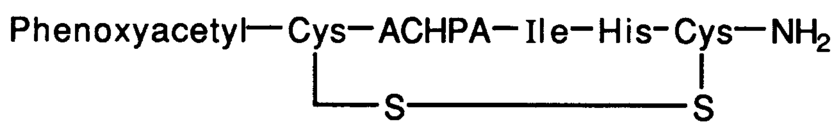

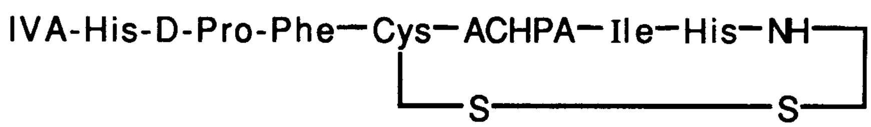

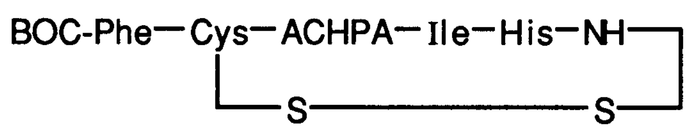

Boger et al., U.S. 4,477,440, discloses renin inhibitor compounds such as

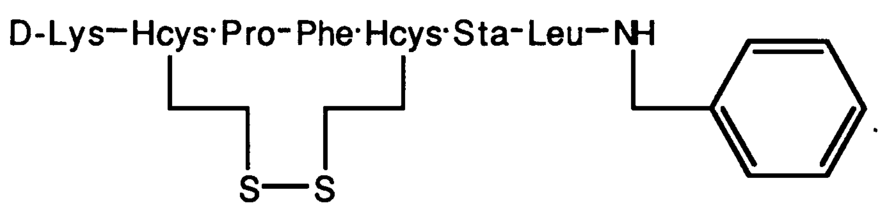

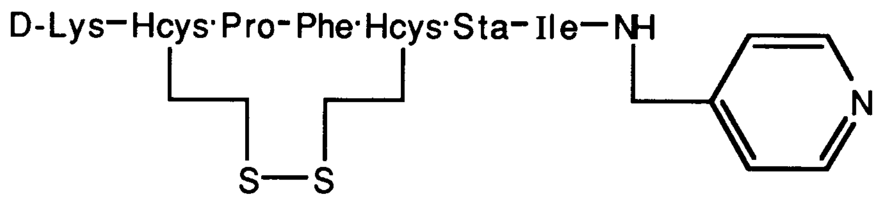

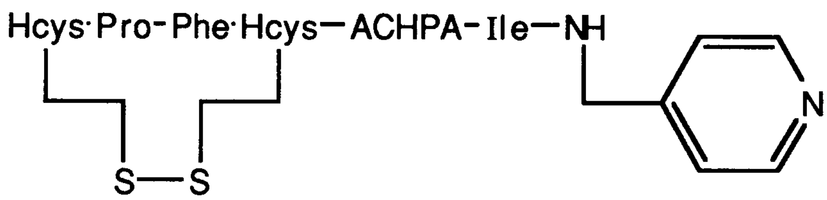

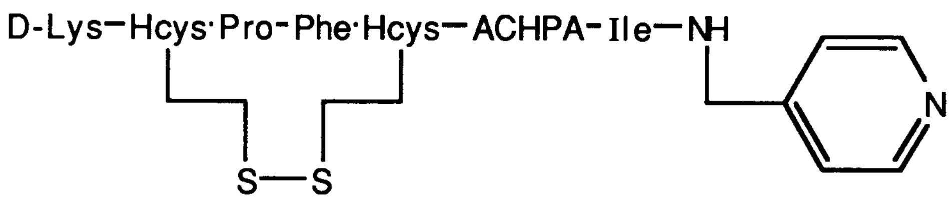

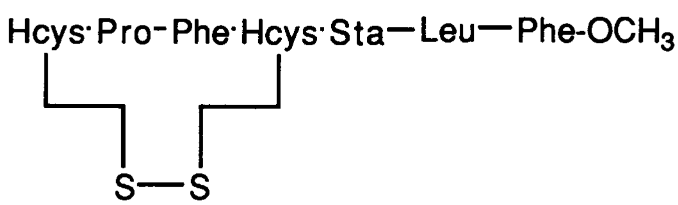

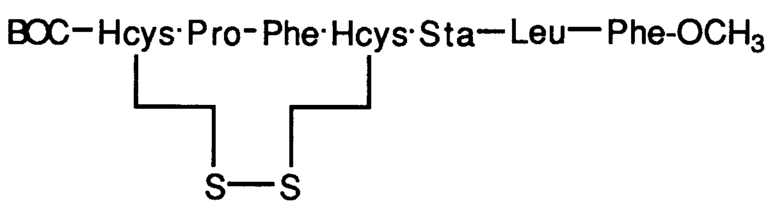

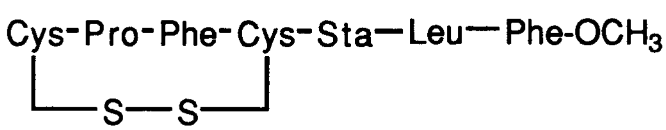

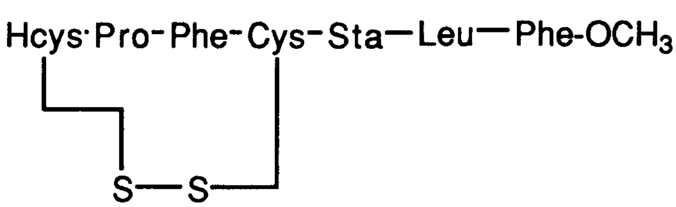

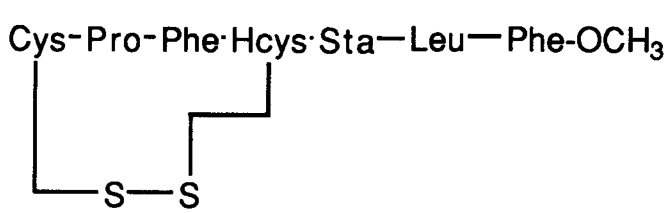

Boger et al., U.S. 4,470,971, discloses renin inhibitor compounds such as
iso-Butyryl-His-Pro-Phe-His-Sta-Val-His-Gly-$NH_2$
iso-Butyryl-His-Pro-Phe-His-Sta-Ile-His-$NH_2$
tert-Butyloxycarbonyl-Phe-His-Sta-Ile-His-$NH_2$
Benzyloxycarbonyl-Phe-His-Sta-Ile-His-$NH_2$
iso-Valeryl-His-Pro-Phe-His-Sta-Ile-His-$NH_2$
iso-Valeryl-His-Pro-Phe-His-Sta-Leu-His-$NH_2$
A preferred compound within this class is IVA-His-Pro-Phe-His-Sta-Ile-His-$NH_2$.

Cazaubon et al., U.S. 4,481,192, discloses renin inhibitor comounds such as BOC-Phe-His-Sta-Ala-Sta-OMe.

Hansen, Jr. et al., U.S. 4,510,085, discloses compounds of the formula

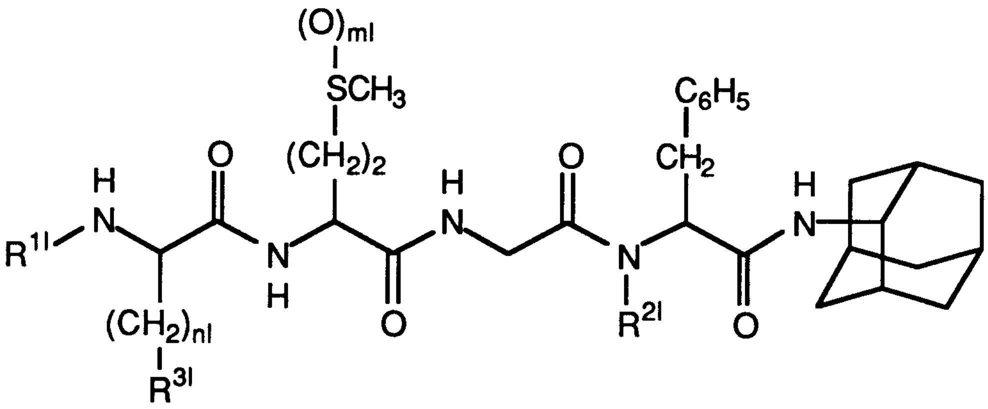

as having renin inhibitory activity
wherein $R^{1l}$ is:
(a) hydrogen; or
(b) alkyl of 1 to 6 carbon atoms, inclusive;
wherein $R^{2l}$ is:
(a) hydrogen; or
(b) alkyl of 1 to 6 carbon atoms, inclusive;
wherein $R^{3l}$ is:

(a)

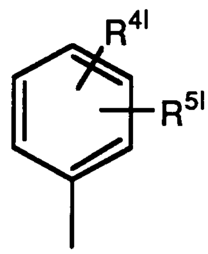

(b)

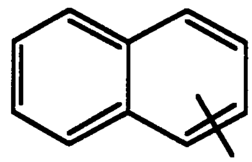

(c)

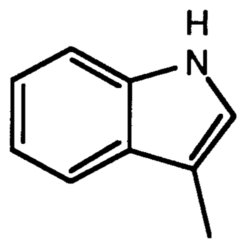

wherein $R^{4l}$ and $R^{5l}$ each being the same or different, are:

(a) hydrogen;

(b) alkyl of 1 to 6 carbon atoms; or

(c) halogen;

wherein ml is 0, 1 or 2,

wherein nl is 0 or an integer of from 1 to 4; and the pharmaceutically acceptable salts thereof.

U.S. 4,514,332 discloses renin inhibitory activity for tetrapeptide adamantyl amides of the formula

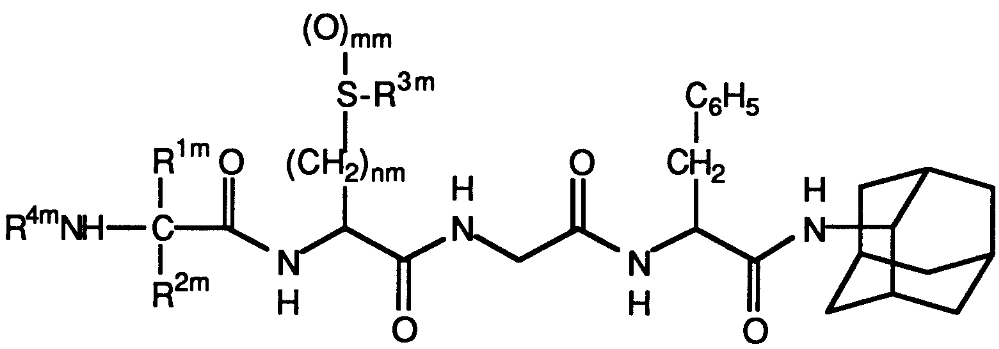

wherein $R^{1m}$ is:

(a)

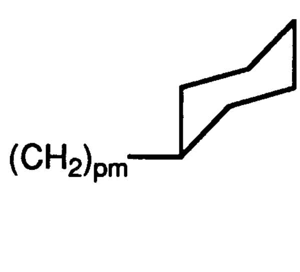

or

(b) straight or branched chain alkyls of 1 to 6 carbon atoms, inclusive;

wherein $R^{2m}$ is:

(a) hydrogen; or

(b) alkyl of 1 to 3 carbon atoms;

wherein $R^{3m}$ is:

(a) $CH_2C_6H_5$; or

(b) alkyl of 1 to 3 carbon atoms;

wherein $R^{4m}$ is:

(a) hydrogen; or

(b) alkyl of 1 to 6 carbon atoms;

wherein $m^m$ is 0, 1 or 2;

wherein $n^m$ is 1 or 2;

wherein $p^m$ is 0, 1 or 2;

and the stereochemical configuration of each of the optically active amino acid residues may independently be D, L or DL; and the pharmacologically acceptable salts thereof.

Castro et al., U.S. 4,185,096, discloses renin inhibitor compounds such as

(I)q

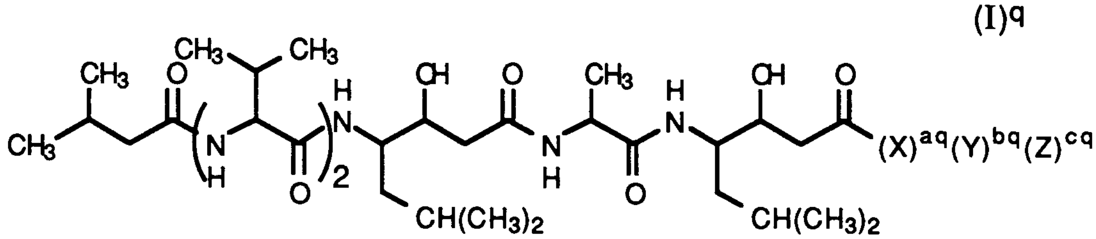

as having renin inhibitory activity.

In the general formula $(I)^q$ each of the symbols $X^{aq}$, $Y^{bq}$ and$Z^{cq}$ which are identical or different, represent an amino-acid residue selected from arginine, glutamic acid, aspartic acid, lysine, histidine or valine, forming with the free carboxyl group of the pepstatin or of the adjacent amino-acid a peptide bond -CONH-; the carboxyl function of the terminal amino-acid may exist in free form or in the form of an ester of an aliphatic alcohol containing 1 to 4 carbon atoms, and the indices aq, bq, and cq are each equal to zero or 1, the sum aq + bq + cq being equal to 1, 2 or 3.

Hayashi et al., U.S. 3,985,875, discloses renin inhibitors of the formula

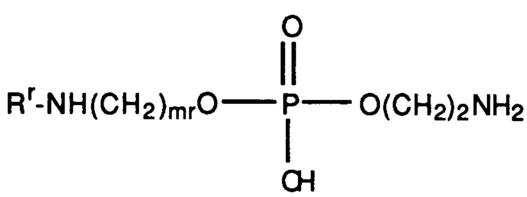

wherein $R^r$ is an octadeca-9,12-dienoyl, octadeca-9,12,15-trienoyl, 4-(4'-chlorophenoxy)phenoxyacetyl or α-[4-(4'-chlorophenoxy)phenoxy]-propionyl group, and $m^r$ is 2 or3, or their pharmaceutically acceptable acid addition salts.

The following definitions apply throughout the specification: IBU = Iso-butyryl; BOC = Tert-butyloxycarbonyl; AHPPA = (3S,4S)-4-amino-3-hydroxy-5-phenylpentanoic acid; ACHPA = (3S,4S)-4-amino-5-cyclohexyl-3-hydroxypentanoic acid; IVA= Iso-valeryl; DAB = 2S-amino-4-aminobutyric acid; HLys = homolysine, 2S-amino-6-aminoheptanoic acid; POA = phenoxyacetyl; Hcys = L-homocysteine.

The above descriptions of classes of renin inhibitors for use in the present invention were taken from the noted patents and publications or abstracts thereof. Reference should be made to such patents and publications themselves for their full disclosures of such classes and specific compounds within such classes, and as to any typographical errors or the like which may have occurred in transcription. Also, in describing such renin inhibitors, the superscript letters a,b,l,m,q and r were included to distinguish among the various classes of compounds and the variable substituent groups thereof.

The antihypertensive effects of NEP inhibitors, A II antagonists and renin inhibitors, and of combinations

of NEP inhibitors with A II antagonists or renin inhibitors are determined according to the following procedures.

For the DOCA salt hypertension model, male Sprague Dawley rats weighing 100-150 g are anesthetized with ether and the right kidney is removed. Three pellets containing Doc acetate (desoxycorticosterone acetate, DOCA, 25 mg/pellet) are implanted subcutaneously. Animals recover from surgery, are maintained on normal rat chow and are allowed free access to a fluid of 1% NaCl and 0.2% KCl instead of tap water for a period of 25-30 days. This procedure results in a sustained elevation in blood pressure and is a slight modification of published procedures (e.g. Brock et al, 1982) that have been used to produce DOCA salt hypertension in the rat.

On the day of the study, animals are again anesthetized with ether and the caudal artery is cannulated for blood pressure measurement. Patency of the caudal artery cannula is maintained with a continuous infusion of dextrose in water at a rate of 0.2 ml/hr. Animals are placed into restraining cages where they recover consciousness. Blood pressure is measured from caudal artery catheter using a Statham pressure transducer attached to a Beckman oscillographic recorder. In addition, a cardiovascular monitoring device (Buxco Eletronics, Inc.) and a digital computer are used to calculate average blood pressures.

After an equilibration period of at least 1.5 hr., animals are dosed subcutaneously (1 ml/kg) with vehicle (methylcellulose, hereinafter MC), NEP inhibitor, A II antagonist, renin inhibitor, a combination of NEP inhibitor and A II antagonist , or a combination of NEP inhibitor and renin inhibitor and blood pressure is monitored for the next 4 hours. The doses of drug are chosen based on amounts previously determined to be effective for inhibition of the respective enzymes.

Two kidney, 1-clip (2K,1C) Goldblatt hypertension is produced in male Sprague Dawley rats as described by DeForrest et al (1984). Rats weighing 180-200 g are anesthetized with ether or Brevital (50 mg/kg, ip) and the left kidney is exposed through a flank incision. A silver clip with an internal diameter of 0.15 mm is placed around the left renal artery. The contralateral kidney remains untouched. The animal is used 3-4 weeks after surgery when sustained hypertension greater than 150 mm Hg is established.

On the day of the experiment, fasted rats are anesthetized with ether and the abdominal aorta is cannulated via the caudal artery with polyethylene tubing. The rats are placed in plastic restrainers and allowed to regain consciousness for at least 90 min. The rats are dosed by oral gavage with a single drug or with a combination of drugs as suspensions in 0.4% methylcellulose vehicle. Blood pressure is recorded continuously from the caudal artery on an oscillographic recorder.

The antihypertensive effect in SHR is determined as follows. Animals are prepared for blood pressure measurement as described above. After stabilization, animals are dosed subcutaneously with test drugs, combinations thereof or placebo and blood pressure is monitored for the next 4 hours.

ANF has been shown to exert beneficial hemodynamic and renal actions in congestive heart failure (CHF) with the exception of the most severe states, in which its actions may be blunted. ANF and the renin angiotensin system also act as physiological antagonists of one another in CHF. Therefore, it is contemplated that the combination of an ANF-potentiating NEP inhibitor and an inhibitor of the renin angiotensin system will be useful in the treatment of CHF. Measurements of the degree of diuresis and natriuresis, as well as hemodynamics, are used to determine the efficacy of the present combination in the treatment of CHF.

The combinations of this invention comprise an NEP inhibitor and an A II antagonist, and an NEP inhibitor and a renin inhibitor. The components of each combination can be administered in the same pharmaceutical composition or by co-administration of separate pharmaceutical compositions. A variety of pharmaceutical dosage forms are suitable, preferably for oral or parenteral administration, although mechanical delivery systems such as transdermal dosage forms are also contemplated.

The daily dosages of the combinations of this invention for treatment of hypertension or congestive heart failure are as follows: for NEP inhibitors, the typical dosage is about 0.3 mg/kg to about 100 mg/kg of mammalian weight per day administered in single or divided doses; for A II antagonists, the typical dosage is about 0.1 mg/kg to about 50 mg/kg of mammalian weight per day administered in single or divided doses; and for renin inhibitors, the typical daily dosage is about 0.1 mg/kg to about 100 mg/kg mammalian weight, administered in single or divided doses. The exact dose of any component or combination to be administered is determined by the attending clinician and is dependent on the potency of the compound administered, the age, weight, condition and response of the patient.

Generally, in treating humans having hypertension or congestive heart failure, the combinations of this invention can be administered in dosage ranges as follows: for the combination of NEP inhibitor and A II antagonist, about 10 to about 500 mg NEP inhibitor per dose given 1 to 4 times a day, and about 5 to about 100 mg A II antagonist given 1 to 3 times a day; and for the combination of NEP inhibitor and renin inhibitor, about 10 to about 500 mg NEP inhibitor given 1 to 4 times a day, and about 5 to about 600 mg

renin inhibitor given 1 to 3 times a day. Where the components of a combination are administered separately, the number of doses of each component given per day may not necessarily be the same, e.g., where one component may have a greater duration of activity, and will therefore need to be administered less frequently.

Typical oral formulations include tablets, capsules, syrups, elixirs and suspensions. Typical injectable formulations include solutions and suspensions.

The typical pharmaceutically acceptable carriers for use in the formulations described above are exemplified by: sugars such as lactose, sucrose, mannitol and sorbitol; starches such as cornstarch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate; polyvinylpyrrolidone; polyvinyl alcohol; stearic acid; alkaline earth metal stearates such as magnesium stearate and calcium stearate; stearic acid; vegetable oils such as peanut oil, cottonseed oil, sesame oil, olive oil and corn oil; non-ionic, cationic and anionic surfactants; ethylene glycol polymers; betacyclodextrin; fatty alcohols; and hydrolyzed cereal solids, as well as other non-toxic compatible fillers, binders, disintegrants, buffers, preservatives, antioxidants, lubricants, flavoring agents, and the like commonly used in pharmaceutical formulations.

Since the present invention relates to treatment of hypertension and congestive heart failure with combinations of active ingredients wherein said active ingredients can be administered separately, the invention also relates to combining separate pharmaceutical compositions in kit form. That is, two kits are contemplated, each combining two separate units: an NEP inhibitor pharmaceutical composition and and A II antagonist pharmaceutical composition in one kit, and an NEP inhibitor pharmaceutical composition and a renin inhibitor pharmaceutical composition in a second kit. The kit form is particularly advantageous when the separate components must be administered in different dosage forms (e.g. oral NEP formulation and parenteral A II antagonist formulation) or are administered at different dosage intervals.

## Claims

**1.** A pharmaceutical composition for treating hypertension or congestive heart failure comprising an effective amount of a combination of a neutral endopeptidase inhibitor and either a renin inhibitor or an angiotensin II antagonist, in a pharmaceutically acceptable carrier.

**2.** A composition of claim 1 wherein:

the neutral endopeptidase inhibitor is selected from the group consisting of N-[N-[1(S)-carboxyl-3-phenylpropyl]-(S)-phenylalanyl]-(S)-isoserine; N-[N-[((1S)-carboxy-2-phenyl)ethyl]-(S)-phenylalanyl]-$\beta$-alanine; N-[2(S)-mercaptomethyl-3-(2-methylphenyl)propionyl]methionine; SQ 28603; UK 69578; thiorphan; retro-thiorphan; phosphoramidon; SQ 29072; and the pro-drugs thereof;

the angiotensin II antagonist is selected from the group consisting of saralasin; sar 1; ile 8 angiotensin II; Dup 753; EXP 6155; EXP 6803; and PD 123319; and

the renin inhibitor is selected from the group consisting of enalkrein; RO 42-5892; A 65317; CP 80794; ES 1005; ES 8891; SQ 34017; CGP 29287; CGP 38560; SR 43845; U-71038; A 62198; and A 64662.

**3.** A kit comprising in separate containers in a single package pharmaceutical compositions for use in combination to treat hypertension or congestive heart failure in mammals which comprises in one container a pharmaceutical composition comprising a neutral endopeptidase inhibitor, and in a second container a pharmaceutical composition comprising a renin inhibitor or an angiotensin II antagonist.

**4.** The use of a neutral endopeptidase (NMEP) inhibitor, in combination with either a renin inhibitor or an angiotensin II antagonist, for the preparation of a pharmaceutical composition useful in the treatment of hypertension or congestive heart failure.

**5.** The use according to claim 4, wherein:

the neutral endopeptidase inhibitor is selected from the group consisting of N-[N-[1(S)-carboxyl-3-phenylpropyl]-(S)-phenylalanyl]-(S)-isoserine; N-[N-[((1S)-carboxy-2-phenyl)ethyl]-(S)-phenylalanyl]-$\beta$-alanine; N-[2(S)-mercaptomethyl-3-(2-methylphenyl)propionyl]methionine; SQ 28603; UK 69578; thiorphan; retro-thiorphan; phosphoramidon; SQ 29072; and the pro-drugs thereof;

the angiotensin II antagonist is selected from the group consisting of saralasin; sar 1; ile 8 angiotensin II; Dup 753; EXP 6155; EXP 6803; and PD 123319; and

the renin inhibitor is selected from the group consisting of enalkrein; RO 42-5892; A 65317; CP 80794; ES 1005; ES 8891; SQ 34017; CGP 29287; CGP 38560; SR 43845; U-71038; A 62198; and A 64662.

**6.** A composition according to any of claims 1 or 2, wherein:
the NMEP inhibitor is administered at a dosage level of 0.3 mg/kg mammalian weight per day;
the angiotensin II antagonist is administered at a dosage level of 0.1 mg/kg to 50 mg/kg mammalian weight per day; and
the renin inhibitor is administered at a dosage level of 0.1 mg/kg to 100 mg/kg mammalian weight per day.

**7.** A process for the preparation of a pharmaceutical composition according to any of claims 1, 2 or 6, which comprises mixing a NMEP inhibitor, in combination with either a renin inhibitor or an angiotensin II antagonist, with a pharmaceutically acceptable carrier.

**8.** A method of treating hypertension or congestive heart failure comprising administering an effective amount of a combination of a neutral endopeptidase inhibitor and either a renin inhibitor or an angiotensin II antagonist to a mammal in need of such treatment.

**9.** A method of claim 8 wherein:
the neutral endopeptidase inhibitor is selected from the group consisting of N-[N-[1(S)-carboxyl-3-phenylpropyl]-(S)-phenylalanyl]-(S)-isoserine; N-[N-[((1S)-carboxy-2-phenyl)ethyl](S)-phenylalanyl]-$\beta$-alanine; N-[2(S)-mercaptomethyl-3-(2-methylphenyl)propionyl]methionine; SQ 28603; UK 69578; thiorphan; retro-thiorphan; phosphoramidon; SQ 29072; and pro-drugs thereof;
the angiotensin II antagonist is selected from the group consisting of saralasin; sar 1; ile 8 angiotensin II; Dup 753; EXP 6155; EXP 6803; and PD 123319; and
the renin inhibitor is selected from the group consisting of enalkrein; RO 42-5892; A 65317; CP 80794; ES 1005; ES 8891; SQ 34017; CGP 29287; CGP 38560; SR 43845; U-71038; A 62198; and A 64662.